# FASCICULE DE BREVET EUROPEEN

(11) **EP 2 986 758 B1**
(45) Date de publication et mention de la délivrance du brevet: **05.06.2019**
(21) Numéro de dépôt: 14729945.7
(22) Date de dépôt: 16.04.2014
(51) Int. Cl.: C12P 7/40, C12P 7/64, C12P 39/00, C25B 3/10, C25B 15/08, C07C 9/15

(54) **PROCÉDÉ DE PRODUCTION D'HYDROCARBURES**
VERFAHREN ZUR HERSTELLUNG VON KOHLENWASSERSTOFFEN
METHOD FOR PRODUCING HYDROCARBIDES

(30) Priorité: 19.04.2013 FR 1353567
(43) Date de publication de la demande: 24.02.2016
(73) Titulaire: Bio-Think, 45170 Chilleurs Aux Bois (FR)
(72) Inventeur: DUPRE, Jean-Yves, F-94300 Vincennes (FR); RODRIGUEZ, Oswaldo Yagüez, F-86240 Smarves (FR)
(74) Mandataire: Aquinov
(86) Numéro de dépôt international: PCT/FR2014/050923
(87) Numéro de publication internationale: WO 2014/170603

(56) Documents cités:
- US-A- 3 992 268
- US-A1- 2011 111 475
- FELICITY A RODDICK ET AL: "Production of Hexanoic Acid by Free and Immobilised Cells of Megasphaera elsdenii: Influence of in-situ Product Removal Using Ion Exchange Resin", J. CHEM. TECH. BIOTECHNOL, vol. 69, no. 3, 1 juillet 1997 (1997-07-01), pages 383-391, XP055094849, DOI: 10.1002/(SICI)1097-4660(199707)69:3<383::A ID-JCTB723>3.0.CO;2-H
- BYUNG-CHUN KIM ET AL: "Production of C4-C6 for Bioenergy and Biomaterials", APPL. CHEM. ENG, vol. 22, no. 5, 1 octobre 2011 (2011-10-01), pages 447-452, XP055094886,
- P F LEVY ET AL: "LIQUID FUELS PRODUCTION FROM BIOMASS", Department of Energy (DOE), Technical reports, 1 janvier 1980 (1980-01-01), pages 01-03, ii-iv, 1-43, XP055094875, Extrait de l'Internet: URL:http://www.osti.gov/scitech/servlets/p url/5276700 [extrait le 2014-01-06]
- KIEUN CHOI ET AL: "In Situ Biphasic Extractive Fermentation for Hexanoic Acid Production from Sucrose by Megasphaera elsdenii NCIMB 702410", APPLIED BIOCHEMISTRY AND BIOTECHNOLOGY, vol. 171, no. 5, 11 novembre 2013 (2013-11-11), pages 1094-1107, XP055095021, ISSN: 0273-2289, DOI: 10.1007/s12010-013-0310-3
- MATTHEW T. AGLER ET AL: "Chain elongation with reactor microbiomes: upgrading dilute ethanol to medium-chain carboxylates", ENERGY & ENVIRONMENTAL SCIENCE, vol. 5, no. 8, 1 janvier 2012 (2012-01-01) , page 8189, XP055095030, ISSN: 1754-5692, DOI: 10.1039/c2ee22101b

## Description

La présente invention concerne un procédé de production d'hydrocarbures à partir de diverses biomasses. Les hydrocarbures obtenus sont notamment destinés à être utilisés comme biocarburants ou biocombustibles.

Depuis plusieurs années, de nombreux pays cherchent des solutions pour remplacer le pétrole qui tend à se raréfier et présente de nombreux inconvénients d'ordre économique et écologique.

Des biocarburants dits de première génération ont été proposés dans un premier temps, mais ceux-ci sont aujourd'hui très critiqués car ils entrent en concurrence directe avec l'alimentation et leurs performances pour la lutte contre l'effet de serre sont contestées du fait du relargage de CO₂ lié au changement d'affectation des terres indirect pour continuer d'alimenter la population mondiale.

L'utilisation de biométhane produit à partir de fermentation anaérobie a également été développée, mais elle nécessite la mise en place de systèmes de distribution très coûteux et de véhicules spécifiquement équipés pour fonctionner au gaz naturel.

D'autres biocarburants partent de sucres ou de matières grasses dont on élimine l'oxygène pour obtenir des hydrocarbures, par des procédés d'hydrogénation qui sont coûteux car ils nécessitent de grandes quantités d'hydrogène.

L'objectif de la présente invention est de proposer une solution palliant ces inconvénients, grâce à des produits qui soient utilisables dans tous les moteurs existants et qui entrent dans la catégorie des biocarburants de deuxième et troisième générations.

A cet effet, l'invention propose un procédé qui consiste à produire des acides gras volatils par fermentation anaérobie à partir de différentes biomasses, et à transformer ces acides gras volatils en hydrocarbures par électrolyse.

La production d'hydrocarbures par électrolyse d'acides gras volatils est connue depuis plus de trente ans. On peut citer à cet effet par exemple le brevet français FR2303854 et le brevet américain US3992268. Toutefois, à ce jour, ce procédé n'a jamais été mis en oeuvre industriellement, car il ne fonctionne avec de bons rendements massiques et énergétiques qu'avec une proportion élevée (de l'ordre de 90%) d'acides ayant au moins 5 à 6 atomes de carbone (acide valérique et acide caproïque) avec surtout une proportion élevée (supérieure à 50%) d'acides à plus de 6 atomes de carbone, alors que la fermentation anaérobie produit majoritairement des acides gras volatils plus courts (l'acide caproïque n'est en général que de quelques pour cents et dans les travaux menés par LEVY, Liquid fuels production from biomass, Dynatech 1980, cette proportion, même après ajout d'acétate, ne dépassait pas 12%).

Plus récemment, il a été décrit la possibilité d'élongation des acides gras volatils par fermentation par une bactérie particulière, *Clostridium kluyveri* (Matthew T. Agler et al : chain élongation with reactor microbiomes : upgrading dilute éthanol to médium chain carboxylates Energy and Environmental sciences 2012). Néanmoins ce procédé nécessite l'utilisation d'éthanol ou d'hydrogène en grande quantité si bien qu'il n'est pas viable économiquement, les produits obtenus étant trop coûteux.

La publication de brevet US2011/111475 A1 décrit un procédé de formation d'hydrocarbures par electrolyse d'acides gras volatils (VFA) obtenus par fermentation anaérobie de biomasse, caractérisé en ce qu'il utilise des microorganismes du rumen. Le document indique d'utiliser des bactéries du genre Clostridium ou Butyrvibrio pour augmenter le taux d'acide butyrique et caproïque dans une deuxième étape de fermentation, suivant l'étape initiale de fermentation produisant principalement l'acide acétique et propionique.

On sait par ailleurs qu'il est possible d'obtenir à partir des sucres alimentaires courants (glucose, sucrose) de l'acide caproïque mais compte tenu du coût actuel des sucres des plantes saccharifères (de l'ordre de 1000 dollars par tonne) et du rendement massique final des réactions (une tonne d'hydrocarbures pour environ 4 tonnes de sucre), il est tout à fait improbable que l'on puisse produire économiquement des hydrocarbures à partir de tels sucres.
Pour pallier ces inconvénients, la présente invention propose un procédé comprenant au moins les étapes suivantes :
- a) fermentation anaérobie d'une matière première fermentescible pour obtenir des acides gras volatils,
- b) élongation des acides gras volatils obtenus à l'étape a) par fermentation avec une ou plusieurs bactéries du genre *Megasphaera,* encore plus préférentiellement avec la bactérie *Megasphaera elsdenii,* et extraction de ces acides du moût de fermentation, les acides gras obtenus à cette étape étant majoritairement des acides gras avec une chaîne à au moins 5 atomes de carbone,
- c) obtention d'hydrocarbures en soumettant les acides gras obtenus à l'étape b) à une électrolyse de Kolbe.
Avantageusement la fermentation des sucres et des acides gras volatils par une bactérie du genre *Megasphaera,* et notamment *Megasphaera elsdenii,* permet d'obtenir majoritairement des acides gras avec une chaîne à au moins 5 atomes de carbone, sans nécessiter la présence obligatoire d'éthanol ou d'hydrogène.

Le procédé est tout à fait compatible avec une production industrielle, à la fois en termes économiques et grâce à sa simplicité de réalisation.

Les hydrocarbures obtenus peuvent être utilisés comme biocarburants en mélange à l'essence, au gazole ou au kérosène, ou comme biocombustibles.

Selon un autre avantage, les matières premières susceptibles d'être utilisées sont nombreuses parmi les matières premières préconisées pour les biocarburants de deuxième génération et de troisième génération.

D'autres caractéristiques et avantages ressortiront de la description en détail qui va suivre de l'invention.

L'invention vise donc un procédé de production d'hydrocarbures, comprenant au moins la mise en oeuvre des étapes suivantes :
- a) fermentation anaérobie d'une matière première fermentescible pour obtenir des acides gras volatils,
- b) élongation des acides gras volatils obtenus à l'étape a) par fermentation avec une ou plusieurs bactéries du genre *Megasphaera,* encore plus préférentiellement avec la bactérie *Megasphaera elsdenii,* et extraction de ces acides du moût de fermentation, les acides gras obtenus à cette étape étant majoritairement des acides gras avec une chaîne à au moins 5 atomes de carbone,
- c) obtention d'hydrocarbures en soumettant les acides gras obtenus à l'étape b) à une électrolyse de Kolbe.

La première étape consiste à fermenter une biomasse par fermentation anaérobie pour obtenir des acides gras volatils.

Par matière première fermentescible ou biomasse, on entend toute matière première susceptible de produire des acides gras volatils par fermentation.

Préférentiellement les matières premières sont choisies parmi les plantes ligno-cellulosiques ainsi que les déchets et sous-produits de ces plantes, les matières d'origine animale, en particulier les farines et matières grasses animales, les algues ou sous-produits des algues et les déchets et effluents organiques.

Les matières premières peuvent être pré-traitées par un acide, une base, un solvant, un procédé physique ou des enzymes avant mise en oeuvrede l'étape a).

De façon particulièrement adaptée, les matières premières sans pré-traitement ou après pré-traitement, sont choisies parmi :
- les sous-produits (mélasse, vinasse, égouts de sucrerie, drèches) des plantes sucrières ou amidonnières, comme par exemple la betterave, la canne à sucre ou des céréales,
- les sucres obtenus par hydrolyse(s) de biomasse d'origine terrestre (bois, taillis à courte rotation, paille, tiges) préférentiellement précédée(s) d'une délignification,
- des plantes terrestres récoltées « plante entière », comme par exemple le maïs ou le sorgho,
- les sucres obtenus par hydrolyse de biomasse d'origine aquatique, comme des micro-algues, des macro-algues ou des jacinthes d'eau,
- des effluents ou déchets industriels, urbains, agricoles ou d'élevage.

Avantageusement les microorganismes utilisés pour la fermentation anaérobie des matières premières permettent d'assurer la première phase d'hydrolyse de la cellulose et des hémicelluloses en faisant appel à des pré-traitements simples sans avoir nécessairement à utiliser des enzymes coûteuses comme c'est le cas dans de nombreux procédés existants.

L'étape a) est préférentiellement réalisée par des pools de bactéries, comme c'est le cas pour les fermentations qui produisent du biogaz ou des bactéries qui produisent des acides gras volatils courts (*Clostridium Thermoacéticum, Tyrobutyricum* ou *Propionibactérium* par exemple) seules ou en mélange. Il peut s'agir en particulier de fermentations mésophiles (vers 37°C) ou thermophiles (vers 60-70°C).

Les acides gras volatils obtenus sont majoritairement des acides courts : acétique, propionique et butyrique.

L'étape b) du procédé selon l'invention consiste à allonger la chaîne carbonée de ces acides gras volatils obtenus à l'étape a) par fermentation avec une ou plusieurs bactérie(s) du genre *Megasphaera,* encore plus préférentiellement avec la bactérie *Megasphaera elsdenii.*

Par bactérie du genre *Megasphaera* (ou la bactérie *Megasphaera elsdenii*) au sens de l'invention, on entend également les bactéries recombinantes incorporant des gènes d'une bactérie du genre *Megasphaera,* (ou les bactéries recombinantes incorporant des gènes de la bactérie *Megasphaera elsdenii*).

Les bactéries du genre *Megasphaera* peuvent notamment être des bactéries sauvages provenant du rumen ou d'effluents de ruminants ou de résidus de procédés industriels tels que ceux de l'industrie de la bière, ou des bactéries obtenues dans des banques de souches de micro-organismes. Outre *Megasphaera elsdenii,* on peut citer également *Megasphaera cerevisae.*

L'étape b) peut être réalisée sous plafond ou bullage de méthane.

Le terme « sous plafond » de gaz, signifie que le gaz est présent au-dessus de la phase liquide.

Le terme « sous bullage » de gaz, signifie que le gaz est introduit sous forme de bulles dans le liquide.

L'étape b) peut être également réalisée :
- sous plafond ou bullage de biogaz,
- sous plafond ou bullage d'un mélange de méthane, de gaz carbonique et d'hydrogène dans des proportions variables,
- en présence dans le liquide fermentaire d'un additif inhibiteur de la co-enzyme M-réductase, tel que le 2bromoéthansulfonate (BES) ou tout autre inhibiteur de la co-enzyme M-réductase connu de l'homme du métier.

Le plafond ou bullage de gaz ou la présence d'un inhibiteur de la co-enzyme M-réductase, permet d'améliorer le procédé en accélérant la vitesse d'utilisation des sucres et des acides gras courts et en évitant la destruction des acides gras allongés dans le milieu et leur retour à des acides gras plus courts.

L'étape b) peut également éventuellement être réalisée en présence d'éthanol.

Pour conduire à une bonne efficacité de l'élongation et éviter que les enzymes de la bactérie ne détruisent les acides gras moyens qui ont été formés, l'étape b) doit préférentiellement être réalisée dans des conditions spécifiques, en particulier un pH compris entre 5 et 6,5, et un potentiel d'oxydo-réduction très bas, notamment compris entre -500 mV et -600 mV. L'étape b) d'élongation avec *Megasphaera* est essentielle et doit permettre avant extraction d'obtenir des acides gras dont au moins 40% sont des acides gras avec une chaîne à 6 atomes de carbone.

Très préférentiellement, les étapes a) et b) sont réalisées séparément.

Selon une première variante, les étapes a) et b) peuvent être réalisées ensemble en une seule et même étape en mettant dans un seul fermenteur la matière première fermentescible en co-culture avec un microorganisme capable d'hydrolyser la cellulose et une ou plusieurs bactérie(s) du genre *Megasphaera,* encore plus préférentiellement avec la bactérie *Megasphaera elsdenii*.

Selon une deuxième variante les étapes a) et b) sont réalisées ensemble avec des pools de bactéries auxquelles on a ajouté un innoculum à base d'une bactérie du genre *Megasphaera,* encore plus préférentiellement avec la bactérie *Megasphaera elsdenii*.

Une fois l'élongation réalisée, les acides gras volatils allongés doivent préférentiellement être extraits du moût de fermentation pour mettre en oeuvre l'étape c).

Les acides gras (principalement acétique et butyrique) courts restant dans le moût de fermentation à l'étape b) après extraction des acides gras allongés, peuvent être réutilisés aux étapes a) ou b) ou bien utilisés pour produire des alcènes par la variante Hoefer-Moest de l'électrolyse de Kolbe.

De façon préférée, l'extraction est réalisée de manière continue, hors du fermenteur ou à l'intérieur du fermenteur s'il s'agit d'un fermenteur biphasique, les phases aqueuse et organique étant simplement superposées ou soumises à des actions permettant de renforcer leur interface (agitation, bullage, etc.).

Selon un mode de réalisation particulièrement adapté, cette extraction peut être réalisée avec un extractant et/ou un solvant. L'extractant peut être choisi par exemple parmi les composés organophosphorés tels que le trioctylphosphine oxide et les amines tertiaires ou quaternaires grasses telle que la trioctylamine. Le solvant peut être choisi par exemple parmi les alcools, les esters, les alcanes et des mélanges d'hydrocarbures tels que le kérosène. Préférentiellement l'extractant et le solvant sont utilisés à l'intérieur du fermenteur dans une fermentation bi-phasique.

Selon une autre variante, cette extraction peut être réalisée avec un contacteur membranaire qui permet d'éviter de mettre en contact l'extractant et le solvant avec la bactérie.

Ce mode d'extraction peut préférentiellement être mis en oeuvre en milieu aqueux avec une membrane hydrofuge qui, de manière surprenante, extrait très préférentiellement les acides à 6 atomes de carbone et plus, ce qui permet d'augmenter la concentration en de tels acides.

L'étape b) après extraction conduit à un mélange d'acides gras comportant plus de 50% d'acides à 6 atomes de carbone et plus de 90% d'acides à 5 atomes de carbone et plus, ce qui correspond aux conditions nécessaires pour rendre l'électrolyse de Kolbe économique, les autres acides gras pouvant être recyclés dans l'étape a) de fermentation anaérobie.

L'étape c) consiste ensuite à soumettre les acides gras obtenus à l'étape b) à une électrolyse. Cette électrolyse est connue sous le nom de l'électrolyse de Kolbe.

A l'anode, la réaction est une oxydation qui conduit à la formation du radical acide RCOO^{•}. Puis deux radicaux de ce type se combinent pour « dimériser », ce qui aboutit à la formation de CO₂ et d'un hydrocarbure R-R de longueur de chaîne double de celle de la molécule d'origine diminuée d'un atome de carbone. A la cathode, les ions H+ conduisent à la formation d'hydrogène qui s'échappe sous forme de gaz H₂.

Il existe différentes variantes de cette électrolyse, dont une consiste à faire une dimérisation croisée entre un AGV et des acides gras d'huiles végétales.

Cette électrolyse à partir des acides gras volatils obtenus selon l'invention à l'étape b), permet d'obtenir des hydrocarbures.

Préférentiellement, les hydrocarbures obtenus sont des alcanes avec une chaîne à au moins 8 atomes de carbone, encore plus préférentiellement des alcanes avec une chaîne de 8 à 14 atomes de carbone.

L'étape c) peut être suivie d'une étape d) de purification des hydrocarbures obtenus, selon tout procédé physique ou chimique connu, comme par exemple le lavage à l'eau, le nettoyage par un solvant, la distillation, etc.

Ces hydrocarbures peuvent être utilisés comme biocarburants notamment en mélange à l'essence, au kérosène ou au gazole, ou encore comme biocombustibles.

A l'étape c), une réaction annexe peut avoir lieu à l'anode avec les acides gras volatils courts présents en même temps que les acides à 5 ou 6 atomes de carbone, qui conduit préférentiellement à la formation d'alcènes tels que l'éthylène et le propylène. Ces produits peuvent servir de base à la production de nombreux produits chimiques dont des polymères tels que le polyéthylène et le polypropylène.

Selon un mode de réalisation, le procédé selon l'invention peut également comprendre des étapes permettant de valoriser les co-produits.

L'invention est à présent illustrée par un exemple de mise en oeuvre et des résultats d'essais.

### Etapes a) et b)

### Exemple 1

Un essai de fermentation d'une solution à 4% de saccharose dans l'eau avec la souche NCIMB 702410 de *Megasphaera elsdenii* a été réalisé durant 7 jours à 37°C dans un erlenmyer de 500ml contenant :
- 250ml de milieu biologique (à base d'éléments nutritifs connus : extrait de levure, peptones, extrait de viande) additivé de saccharose ou autres sucres et de 5g/l de butyrate de sodium,
- 2ml d'aliquot de la souche bactérienne,
- 100ml de solvant d'extraction (Alamine 336 à 10% dans une solution d'alcool oléique), et
- au-dessus, un plafond de méthane (>99%) en milieu stérile.

Après 7 jours, la production des AGV a été dosée par chromatographie en phase gazeuse. La composition des phases aqueuse et organique obtenue est présentée dans le tableau 1 ci-après :

**Tableau 1**

| | Phase aqueuse | Phase organique | Total dans les 2 phases En g/l | Profil d'acide pour les 2 phases |
|---|---|---|---|---|
| Acide formique | 0,0 | 0,3 | 0,3 | 1,2% |
| Acide acétique | 0,3 | 0,3 | 0,6 | 2,2% |
| Acide propionique | 0,0 | 0,0 | 0,0 | 0,2% |
| Acide isobutyrique | 0,2 | 0,4 | 0,6 | 2,3% |
| Acide butyrique | 2,9 | 2,6 | 5,5 | 22,1% |
| Acide isovalérique | 0,4 | 1,1 | 1,5 | 5,9% |
| Acide valérique | 1,4 | 4,7 | 6,1 | 24,2% |
| Acide caproïque | 0,8 | 9,8 | 10,6 | 41,9% |

La proportion totale d'acide caproique est de 42%, et la proportion en phase organique est de 51%. La proportion totale d'acides à 5 atomes de carbone et plus (acides valérique, isovalérique et caproïque) dans les deux phases est de 72%. La proportion dans la phase organique est de 81%.

### Exemple 2

Le même essai a été réalisé avec du glucose à la place du saccharose. La proportion totale d'acides à 5 atomes de carbone et plus dans les deux phases est supérieure à 73% et la proportion dans la phase organique est de plus de 90%.

### Exemple 3

Une essai de fermentation d'une solution aqueuse comportant au départ 7,6 g/l d'acide acétique et 5,2 g de glucose avec la souche NCIMB 702410 de *Megasphaera elsdenii* a été réalisé, à 37°C avec un pH régulé à 6 et un potentiel redox inférieur à 500 mV, dans un bioréacteur de volume utile de 2,5 litres équipé d'un contacteur membranaire pour l'extraction/concentration des acides gras. On a rajouté régulièrement du glucose lorsque la concentration descendait au-dessous de 0,2 g/l
Au bout de 112 heures, la quantité totale d'acides gras dans le réacteur et l'extracteur a été au total de 14,2 g/l dont 7,4 g/l d'acide butyrique, 0,2 g/l d'acide valérique et 6,4 g/l d'acide caproïque (45%). La concentration en acide acétique a été ramenée de 7,6 à 2,2 g/l soit une consommation de 5,4 g/l et la consommation de glucose a été de 17,5 g/l.

Il apparaît donc que l'on a obtenu une production significative d'acide caproïque dont le carbone provenait en partie du glucose et en partie de l'acide acétique. Comme une partie de l'acide acétique a également été transformée en acide butyrique, on peut affirmer que l'on a procédé à une réelle élongation de l'acide acétique en acides butyrique et caproïque. Le rendement massique global a été de 56% et la productivité en acide caproïque atteignait 0,10 g/l/h à la fin de la fermentation.

### Exemple 4

Il s'agit d'un essai similaire au précédent. Au bout de 140 heures, 20g/l de glucose et 6,4g/l d'acétate ont été consommés et 8,5 g/l d'acide caproïque et 8,2 g/l d'acide butyrique ont été produits. Il y a donc bien eu une élongation de l'acide acétique en butyrique et caproïque et le rendement massique global a été de 63%.

L'extraction par résines échangeuse d'ions qui a été faite après l'essai a porté très préférentiellement sur l'acide caproïque, puisque la proportion d'acide caproïque obtenue après récupération sur les résines était de 96%. Les acides ainsi obtenus peuvent être facilement utilisés pour l'électrolyse puisqu'ils ne sont pas en solution aqueuse.

Cet essai montre que l'on peut extraire une solution d'acides très concentrée en acide caproïque, apte à donner des rendements élevés en alcanes par l'électrolyse de Kolbe, les acides qui n'ont pas été extraits (majoritairement acides acétique et butyrique) pouvant être recyclés dans le fermenteur pour une nouvelle élongation par *Megasphaera* ou bie récupérés pour produire des alcènes par la variante Hoefer-Moest de l'électrolyse de Kolbe.

### Exemple 5

Ce dernier essai a permis de mettre en oeuvre successivement les étapes a) et b).

300 g (brut) d'épluchures de pomme de terre dans 2 700 ml d'eau ont été soumis à une fermentation anaérobie avec 300ml d'inoculum (digesteur fumier/paille voie sèche). Le fermenteur était régulé à 55°C ainsi qu'à pH 5,8. La méthanogénèse était inhibée par 0,1340g de BES (2-bromoéthane sulfonate). Au bout de 400 heures, les concentrations en AGV courts obtenus à la première étape ou présents au début de la fermentation étaient de 2,2 g/l d'acide acétique, 0,7 g/l d'acide propionique et 1 g/l d'acide butyrique.

Le moût de fermentation après filtration a été soumis à la fermentation par Megashaera par ajout au reliquat de la fermentation précédente après soutirage. Au début de la fermentation, les concentrations étaient de 3 g/l d'acétique, 3,5 g/l de butyrique, 0,2 g/l de valérique et 2,4 g/l de caproïque. Au bout de 120 heures, la quantité totale d'acides gras récupérée, ramenée au volume de fermenteur et d'extracteur, a été de 18 g/l dont 1,2 d'acétique, 8 g/l de butyrique, 0,8 g/l de valérique et 8 g/l de caproïque, La consommation de glucose a été de 19,5 g/l et celle d'acide acétique de 1,8 g/l. On a bien obtenu de nouveau une élongation des acides courts présents initialement ou obtenus à l'étape a) principalement vers le butyrique et le caproïque, avec un rendement massique global de 18 g/l sur 19,5 g/l de glucose plus 9,1 g/l d'AGV initiaux soit 63%.

L'élution à partir des résines a permis d'extraire un mélange comportant 81% d'acide caproïque. Cette composition obtenue est donc très intéressante pour mettre en oeuvre l'électrolyse de Kolbe.

### Etape c)

### Exemple 6

Une cellule d'électrolyse de type filtre-presse a été montée. Elle est composée de deux plaques de graphite usinées pour permettre l'accès de la solution de réactif aux électrodes. Deux électrodes de platine (de 25cm³) ont été placées sur chaque plaque graphite.

80ml de solution d'acide caproïque à 1 mol.L-1 dans NaOH ont été placés dans un tube gradué possédant une sortie et une entrée, puis soumis à l'électrolyse. Cet appareillage permet de mesurer les quantités de solution à ajouter pour maintenir la concentration à un niveau constant, tout en mesurant la tension et l'intensité du courant et la quantité de produits obtenus qui surnagent en haut de la solution.

Les produits détectés sont les suivants :
- 90% de décane,
- 9% de caproate de pentyl, et
- quelques produits provenant d'impuretés présentes dans les réactifs utilisés.

Le rendement massique global est quasiment de 100% du rendement théorique et la consommation électrique a été de 1,75kWh/litre de produit, ce qui représente environ 15% de la valeur énergétique du carburant obtenu.

### Exemple 7

Le même essai a été réalisé avec une solution comportant 75% d'acide caproïque et 25% d'acide valérique. Les produits détectés ont été presque uniquement des alcanes à 8 atomes de carbone. La consommation électrique a été de 2,5kWh/litre de produit, ce qui représente environ 22% de la valeur énergétique du carburant obtenu.

### Exemple 8

Le même essai a été réalisé avec une solution comportant 50% d'acide caproïque, 20% d'acide valérique, 20% d'acide isovalérique, 10% d'acide butyrique (moût de synthèse représentatif du moût que l'on peut aisément obtenir par fermentation par *Megasphaera*). Les produits obtenus ont été à plus de 90% des alcanes à 8 atomes de carbone et plus (octane, nonane, décane). La consommation électrique a été de 2,1 kWh/litre de produit, ce qui représente environ 18% de la valeur énergétique du carburant obtenu.

### Exemple 9

Le même essai a été réalisé avec une solution comportant 70% d'acide caproïque, 20% d'acide valérique, 5% d'acide isovalérique et 5% d'acide butyrique (moût de synthèse). Les produits obtenus ont été quasi uniquement des alcanes à 8 atomes de carbone et plus : octane 6%, nonane et methyl-octane 29%, décane 65%. La consommation électrique a été de 1,7 kWh/litre de produit, ce qui représente environ 15% de la valeur énergétique du carburant obtenu.

De manière inattendue au regard de l'état de l'art, ces essais montrent que le procédé selon l'invention permet d'obtenir une très forte proportion d'alcanes à 8 atomes de carbone et plus utilisables comme carburants en mélange dans l'essence, le kérosène ou le gazole ou comme combustibles. Le procédé est en outre relativement simple, avec des rendements massiques élevés (il faut environ 5 tonnes de biomasse sèche pour une tonne d'hydrocarbures, ce qui est à peine au-dessous du rendement théorique) et une faible consommation d'énergie électrique, permettant ainsi une exploitation économiquement viable et très intéressante.

## Revendications

1. Procédé de production d'hydrocarbures, **caractérisé en ce qu'**il comprend au moins la mise en oeuvre des étapes suivantes :
- a) fermentation anaérobie d'une matière première fermentescible pour obtenir des acides gras volatils,
- b) élongation des acides gras volatils obtenus à l'étape a) par fermentation avec une ou plusieurs bactérie(s) du genre *Megasphaera,* et extraction des acides gras obtenus du moût de fermentation, les acides gras obtenus à cette étape étant majoritairement des acides gras avec une chaîne à au moins 5 atomes de carbone,
- c) obtention d'hydrocarbures en soumettant les acides gras obtenus à l'étape b) à une électrolyse de Kolbe.

2. Procédé de production d'hydrocarbures selon la revendication 1, **caractérisé en ce que** l'étape b) est réalisée par fermentation avec la bactérie *Megasphaera elsdenii*.

3. Procédé de production d'hydrocarbures selon l'une des précédentes revendications, **caractérisé en ce que** l'étape b) est réalisée avec un pH compris entre 5,5 et 6,5.

4. Procédé de production d'hydrocarbures selon l'une des précédentes revendications, **caractérisé en ce que** l'étape b) est réalisée avec un potentiel d'oxydo-réduction compris entre -500 et -600 mV.

5. Procédé de production d'hydrocarbures selon l'une des précédentes revendications, **caractérisé en ce que** l'étape b) de fermentation des acides gras volatils avec une ou plusieurs bactérie(s) du genre *Megasphaera* est réalisée sous plafond ou bullage de méthane, ou sous plafond ou bullage de biogaz ou sous plafond ou bullage d'un mélange de méthane, de gaz carbonique et d'hydrogène dans des proportions variables, ou en présence d'un additif inhibiteur de la co-enzyme M-réductase dans le liquide fermentaire.

6. Procédé de production d'hydrocarbures selon l'une des précédentes revendications, **caractérisé en ce que** l'étape b) est réalisée en présence d'éthanol.

7. Procédé de production d'hydrocarbures selon l'une des précédentes revendications, **caractérisé en ce que** les acides gras obtenus à l'étape b) sont majoritairement des acides gras avec une chaîne de 6 à 8 atomes de carbone.

8. Procédé de production d'hydrocarbures selon l'une des précédentes revendications, **caractérisé en ce que** les acides gras obtenus à l'étape b) sont :
- pour au moins 50% des acides gras avec une chaîne à 6 atomes de carbone, et
- pour au moins 90% des acides gras avec une chaîne à au moins 5 atomes de carbone.

9. Procédé de production d'hydrocarbures selon l'une des précédentes revendications, **caractérisé en ce que** les hydrocarbures obtenus à l'étape c) sont majoritairement des alcanes avec une chaîne à au moins 8 atomes de carbone.

10. Procédé de production d'hydrocarbures selon l'une des précédentes revendications, **caractérisé en ce que** les hydrocarbures obtenus à l'étape c) sont majoritairement des alcanes avec une chaîne de 8 à 14 atomes de carbone.

11. Procédé de production d'hydrocarbures selon l'une des précédentes revendications, **caractérisé en ce que** les étapes a) et b) sont réalisées ensemble en une seule et même étape.

12. Procédé de production d'hydrocarbures selon la revendication 11, **caractérisée en ce que** les étapes a) et b) sont réalisées ensemble en mettant la matière première fermentescible en co-culture avec un microorganisme capable d'hydrolyser la cellulose et une ou plusieurs bactérie(s) du genre *Megasphaera.*

13. Procédé de production d'hydrocarbures selon la revendication 11, **caractérisée en ce que** les étapes a) et b) sont réalisées ensemble avec des pools de bactéries renforcées en une bactérie du genre *Megasphaera.*

14. Procédé de production d'hydrocarbures selon l'une des précédentes revendications, **caractérisé en ce que** les acides gras volatils allongés obtenus à l'étape b) sont extraits du moût de fermentation de manière continue.

15. Procédé de production d'hydrocarbures selon l'une des précédentes revendications, **caractérisé en ce que** les acides gras courts restant dans le moût de fermentation à l'étape b) après extraction des acides gras allongés, sont réutilisés aux étapes a) ou b) ou bien utilisés pour produire des alcènes par la variante Hoefer-Moest de l'électrolyse de Kolbe.

16. Procédé de production d'hydrocarbures selon l'une des précédentes revendications, **caractérisé en ce que** les acides gras volatils allongés obtenus à l'étape b) sont extraits du moût de fermentation avec un extractant et/ou un solvant.

17. Procédé de production d'hydrocarbures selon la revendication 16, **caractérisé en ce que** l'extractant est choisi parmi les composés organophosphorés et les amines tertiaires ou quaternaires grasses, et le solvant est choisi parmi les alcools, les esters, les alcanes et des mélanges d'hydrocarbures.

18. Procédé de production d'hydrocarbures selon la revendication 16 ou 17, **caractérisé en ce que** l'extractant et le solvant sont utilisés à l'intérieur d'un fermenteur bi-phasique.

19. Procédé de production d'hydrocarbures selon l'une des précédentes revendications, **caractérisé en ce que** les acides gras volatils allongés obtenus à l'étape b) sont extraits avec un contacteur membranaire à membrane hydrofuge.

20. Procédé de production d'hydrocarbures selon l'une des précédentes revendications, **caractérisé en ce qu'**il comprend une étape d) de purification des hydrocarbures obtenus à l'étape c).

21. Procédé de production d'hydrocarbures selon l'une des précédentes revendications, **caractérisé en ce que** les matières premières fermentescibles sont pré-traitées avant l'étape a) par un acide, une base, un solvant, un procédé physique ou des enzymes.

22. Procédé de production d'hydrocarbures selon l'une des précédentes revendications, **caractérisé en ce que** les matières premières fermentescibles sont choisies parmi les sous-produits des plantes sucrières ou amidonnières, les sucres obtenus par hydrolyse de biomasse d'origine terrestre éventuellement précédée d'une délignification, et les sucres obtenus par hydrolyse de biomasse d'origine aquatique, des effluents ou déchets industriels, urbains, agricoles ou d'élevage.

## Patentansprüche

1. Verfahren zur Herstellung von Kohlenwasserstoffen, **dadurch gekennzeichnet, dass** dieses die Ausführung wenigstens eines der folgenden Verfahrensschritte umfasst:
- a) anaerobe Fermentierung eines vergärbaren Rohmaterials, um flüchtige Fettsäuren zu erhalten,
- b) Verlängerung der im Verfahrensschritt a) erhaltenen Fettsäuren durch Fermentierung mit einer oder mehreren Bakterie(n) von der Gattung *Megasphaera* und Extraktion der erhaltenen Fettsäuren aus der Fermentierlösung, wobei die in diesem Verfahrensschritt erhaltenen Fettsäuren überwiegend Fettsäuren mit einer Kette aus wenigstens 5 Kohlenstoffatomen sind,
- c) Gewinnung von Kohlenwasserstoffen, indem die im Verfahrensschritt b) erhaltenen Fettsäuren einer Kolbe-Elektrolyse unterzogen werden.

2. Verfahren zur Herstellung von Kohlenwasserstoffen nach Anspruch 1, **dadurch gekennzeichnet, dass** der Verfahrensschritt b) durch Fermentierung mit der Bakterie *Megasphaera elsdenii* ausgeführt wird.

3. Verfahren zur Herstellung von Kohlenwasserstoffen nach einem der vorhergehenden Ansprüche, **dadurch gekennzeichnet, dass** der Verfahrensschritt b) mit einem pH-Wert zwischen 5,5 und 6,5 ausgeführt wird.

4. Verfahren zur Herstellung von Kohlenwasserstoffen nach einem der vorhergehenden Ansprüche, **dadurch gekennzeichnet, dass** der Verfahrensschritt b) mit einem Redoxpotential zwischen -500 und -600 mV ausgeführt wird.

5. Verfahren zur Herstellung von Kohlenwasserstoffen nach einem der vorhergehenden Ansprüche, **dadurch gekennzeichnet, dass** der Verfahrensschritt b) der Fermentierung von flüchtigen Fettsäuren mit einer oder mehreren Bakterie(n) der Gattung *Megasphaera* unter einer Decke oder Blasenbildung von Methan, oder unter einer Decke oder Blasenbildung von Biogas oder unter einer Decke oder Blasenbildung einer Mischung von Methan, Kohlendioxid und Wasserstoff in variablen Anteilen oder in Anwesenheit eines das Coenzym M-Reduktase hemmenden Additivs in der Fermentierflüssigkeit ausgeführt wird.

6. Verfahren zur Herstellung von Kohlenwasserstoffen nach einem der vorhergehenden Ansprüche, **dadurch gekennzeichnet, dass** der Verfahrensschritt b) in Anwesenheit von Ethanol ausgeführt wird.

7. Verfahren zur Herstellung von Kohlenwasserstoffen nach einem der vorhergehenden Ansprüche, **dadurch gekennzeichnet, dass** die im Verfahrensschritt b) erhaltenen Fettsäuren überwiegend Fettsäuren mit einer Kette aus 6 bis 8 Kohlenstoffatomen sind.

8. Verfahren zur Herstellung von Kohlenwasserstoffen nach einem der vorhergehenden Ansprüche, **dadurch gekennzeichnet, dass** die im Verfahrensschritt b) erhaltenen Fettsäuren
- zu wenigstens 50% Fettsäuren mit einer Kette aus 6 Kohlenstoffatomen, und
- zu wenigsten 90% Fettsäuren mit einer Kette aus wenigstens 5 Kohlenstoffatomen
sind.

9. Verfahren zur Herstellung von Kohlenwasserstoffen nach einem der vorhergehenden Ansprüche, **dadurch gekennzeichnet, dass** die im Verfahrensschritt c) erhaltenen Kohlenwasserstoffe überwiegend Alkane mit einer Kette aus wenigstens 8 Kohlenstoffatomen sind.

10. Verfahren zur Herstellung von Kohlenwasserstoffen nach einem der vorhergehenden Ansprüche, **dadurch gekennzeichnet, dass** die im Verfahrensschritt c) erhaltenen Kohlenwasserstoffe überwiegend Alkane mit einer Kette aus 8 bis 14 Kohlenstoffatomen sind.

11. Verfahren zur Herstellung von Kohlenwasserstoffen nach einem der vorhergehenden Ansprüche, **dadurch gekennzeichnet, dass** die Verfahrensschritte a) und b) zusammen in ein und demselben Verfahrensschritt ausgeführt werden.

12. Verfahren zur Herstellung von Kohlenwasserstoffen nach Anspruch 11, **dadurch gekennzeichnet, dass** die Verfahrensschritte a) und b) zusammen ausgeführt werden, indem das vergärbare Rohmaterial in eine Kokultur mit einem zur Hydrolyse von Zellulose geeigneten Mikroorganismus und einer oder mehreren Bakterie(n) der Gattung *Megasphaera* eingebracht wird.

13. Verfahren zur Herstellung von Kohlenwasserstoffen nach Anspruch 11, **dadurch gekennzeichnet, dass** die Verfahrensschritte a) und b) mit um eine Bakterie der Gattung *Megasphaera* verstärkten Bakterienpools zusammen ausgeführt werden.

14. Verfahren zur Herstellung von Kohlenwasserstoffen nach einem der vorhergehenden Ansprüche, **dadurch gekennzeichnet, dass** die im Verfahrensschritt b) erhaltenen, verlängerten flüchtigen Fettsäuren auf kontinuierliche Weise aus der Fermentierungslösung extrahiert werden.

15. Verfahren zur Herstellung von Kohlenwasserstoffen nach einem der vorhergehenden Ansprüche, **dadurch gekennzeichnet, dass** die nach der Extraktion der verlängerten Fettsäuren im Verfahrensschritt b) in der Fermentierlösung verbleibenden kurzen Fettsäuren in den Verfahrensschritten a) oder b) wiederverwendet werden oder dazu verwendet werden, um durch die Hofer-Moest-Abwandlung der Kolbe-Elektrolyse Alkene herzustellen.

16. Verfahren zur Herstellung von Kohlenwasserstoffen nach einem der vorhergehenden Ansprüche, **dadurch gekennzeichnet, dass** die im Verfahrensschritt b) erhaltenen, verlängerten flüchtigen Fettsäuren aus der Fermentierungslösung mit einem Extraktionsmittel und/oder einem Lösungsmittel extrahiert werden.

17. Verfahren zur Herstellung von Kohlenwasserstoffen nach Anspruch 16, **dadurch gekennzeichnet, dass** das Extraktionsmittel aus den Organophosphorverbindungen und den tertiären oder quartären Fettaminen ausgewählt ist und das Lösungsmittel unter den Alkoholen, den Estern, den Alkanen und Kohlenwasserstoffmischungen ausgewählt ist.

18. Verfahren zur Herstellung von Kohlenwasserstoffen nach Anspruch 16 oder 17, **dadurch gekennzeichnet, dass** das Extraktionsmittel und das Lösungsmittel im Inneren eines Zweiphasenfermenters verwendet werden.

19. Verfahren zur Herstellung von Kohlenwasserstoffen nach einem der vorhergehenden Ansprüche, **dadurch gekennzeichnet, dass** die im Verfahrensschritt b) erhaltenen, verlängerten flüchtigen Fettsäuren mit einem Membrankontaktor mit hydrophober Membran extrahiert werden.

20. Verfahren zur Herstellung von Kohlenwasserstoffen nach einem der vorhergehenden Ansprüche, **dadurch gekennzeichnet, dass** dieses einen Verfahrensschritt d) der Reinigung der im Verfahrensschritt c) erhaltenen Kohlenwasserstoffen umfasst.

21. Verfahren zur Herstellung von Kohlenwasserstoffen nach einem der vorhergehenden Ansprüche, **dadurch gekennzeichnet, dass** die vergärbaren Rohmaterialien vor dem Verfahrensschritt a) mit einer Säure, einer Base, einem Lösungsmittel, einem physikalischen Verfahren oder mit Enzymen vorbehandelt werden.

22. Verfahren zur Herstellung von Kohlenwasserstoffen nach einem der vorhergehenden Ansprüche, **dadurch gekennzeichnet, dass** die vergärbaren Rohmaterialien aus den Nebenprodukten der Zuckerpflanzen oder Stärkepflanzen, aus Zuckern, die durch Hydrolyse von vom Land stammender Biomasse, unter Umständen mit vorhergehender Delignifizierung, gewonnen werden, und aus Zuckern ausgewählt werden, die durch die Hydrolyse von aus dem Wasser stammender Biomasse, aus Abwässern, aus industriellen, städtischen, landwirtschaftlichen Abfällen
oder aus der Viehzucht gewonnen werden.

## Claims

1. A method for producing hydrocarbons, **characterised in that** it comprises at least the implementation of the following steps:
- a) anaerobic fermentation of a fermentable raw material in order to obtain volatile fatty acids,
- b) elongation of the volatile fatty acids obtained at step a) by fermentation with one or more bacteria of the *Megasphaera* genus, and extraction of the fatty acids obtained from the fermentation wort, the fatty acids obtained at this step being mainly fatty acids with a chain having at least 5 carbon atoms,
- c) obtaining hydrocarbons by subjecting the fatty acids obtained at step b) to Kolbe electrolysis.

2. A method for producing hydrocarbons according to claim 1, **characterised in that** step b) is performed by fermentation with the *Megasphaera elsdenii* bacterium.

3. A method for producing hydrocarbons according to either of the preceding claims, **characterised in that** step b) is carried out at a pH of between 5.5 and 6.5.

4. A method for producing hydrocarbons according to any of the preceding claims, **characterised in that** step b) is carried out with an oxidation reduction potential of between -500 and -600 mV.

5. A method for producing hydrocarbons according to any of the preceding claims, **characterised in that** step b) of fermentation of volatile fatty acids with one or more bacteria of the *Megasphaera* genus is carried out under a methane ceiling or bubbling, or under biogas ceiling or bubbling or under ceiling or bubbling of a mixture of methane, carbon dioxide and hydrogen in variable proportions, or in the presence of an additive inhibiting the M-reductase coenzyme in the fermentation liquid.

6. A method for producing hydrocarbons according to any of the preceding claims, **characterised in that** step b) is carried out in the presence of ethanol.

7. A method for producing hydrocarbons according to any of the preceding claims, **characterised in that** the fatty acids obtained at step b) are mainly fatty acids with a chain of 6 to 8 carbon atoms.

8. A method for producing hydrocarbons according to any of the preceding claims, **characterised in that** the fatty acids obtained at step b) are:
- to the extent of at least 50%, fatty acids with a chain having 6 carbon atoms, and
- to the extent of at least 90%, fatty acids with a chain having at least 5 carbon atoms.

9. A method for producing hydrocarbons according to any of the preceding claims, **characterised in that** the hydrocarbons obtained at step c) are mainly alkanes with a chain having at least 8 carbon atoms.

10. A method for producing hydrocarbons according to any of the preceding claims, **characterised in that** the hydrocarbons obtained at step c) are mainly alkanes with a chain of 8 to 14 carbon atoms.

11. A method for producing hydrocarbons according to any of the preceding claims, **characterised in that** steps a) and b) are carried out together in a single step.

12. A method for producing hydrocarbons according to claim 11, **characterised in that** steps a) and b) are carried out together by putting the fermentable raw material in co-culture with a microorganism capable of hydrolysing cellulose and one or more bacteria of the *Megasphaera* genus.

13. A method for producing hydrocarbons according to claim 11, **characterised in that** steps a) and b) are carried out together with pools of bacteria reinforced with a bacterium of the *Megasphaera* genus.

14. A method for producing hydrocarbons according to any of the preceding claims, **characterised in that** the elongated volatile fatty acids obtained at step b) are extracted from the fermentation wort in a continuous manner.

15. A method for producing hydrocarbons according to any of the preceding claims, **characterised in that** the short fatty acids remaining in the fermentation wort at step b) after extraction of the elongated fatty acids are reused at steps a) or b) or used to produce alkenes by the Hoefer-Moest variant of Kolbe electrolysis.

16. A method for producing hydrocarbons according to any of the preceding claims, **characterised in that** the elongated volatile fatty acids obtained at step b) are extracted from the fermentation wort with an extraction agent and/or a solvent.

17. A method for producing hydrocarbons according to claim 16, **characterised in that** the extraction agent is chosen from organophosphorus compounds and fatty tertiary or quaternary amines, and the solvent is chosen from alcohols, esters, alkanes and hydrocarbon mixtures.

18. A method for producing hydrocarbons according to claim 16 or 17, **characterised in that** the extraction agent and the solvent are used in a biphase fermenter.

19. A method for producing hydrocarbons according to any of the preceding claims, **characterised in that** the elongated volatile fatty acids obtained at step b) are extracted with a membrane contactor with a hydrophobic membrane.

20. A method for producing hydrocarbons according to any of the preceding claims, **characterised in that** it comprises a step d) of purifying the hydrocarbons obtained at step c).

21. A method for producing hydrocarbons according to any of the preceding claims, **characterised in that** the fermentable raw materials are pretreated before step a) with an acid, a base, a solvent, a physical process or enzymes.

22. A method for producing hydrocarbons according to any of the preceding claims, **characterised in that** the fermentable raw materials are chosen from the by-products of sugary or starchy plants, the sugars obtained by hydrolysis of biomass of terrestrial origin optionally preceded by delignification, and the sugars obtained by hydrolysis of biomass of aquatic origin or industrial, urban, agricultural or animal-husbandry effluent or waste.
